# EUROPEAN PATENT APPLICATION

(11) **EP 0 548 612 A1**
(43) Date of publication of application: **30.06.1993**
(21) Application number: 92120605.8
(22) Date of filing: 03.12.1992
(51) Int. Cl.: A61B 17/34, A61M 25/06

(54) **One-piece injection molded needle bore occlusion device**

(30) Priority: 20.12.1991 US 811475
(71) Applicant: Becton Dickinson and Company, Franklin Lakes, New Jersey 07417-1880 (US)
(72) Inventor: Powell, Melvin J., Edison, New Jersey 08817 (US); Lomba, Luis R., Puerto Rico 00661 (US); Gregg, Joseph J., Hasbrouck Heights, New Jersey 07604 (US)
(74) Representative: Dallmeyer, Georg, Dipl.-Ing.

(57) **Abstract**

A needle assembly (20) includes a cannula (21), having a proximal end and a distal end defining a longitudinal axis and a lumen (22) therethrough, having a distal point (23) and a proximal hub (26). The lumen contains a unitary integrally injection molded device (27) which can function as a stylet or an obturator depending on the design. When the device is designed to function as a stylet, it has a tip formed in the injection molding process which is generally coincident with and occludes the distal point of the cannula. The formed tip of the stylet is held in coaxial alignment with the distal cannula point by a removably conjugate interlocking means (38,39) on the hub of the cannula and the handle of the stylet. When the device is designed to function as an obturator, it has a molded formed rounded point which is contained within the lumen of the needle, the device serving to occlude the bore. The one-piece injection molded device simplifies the manufacturing process by eliminating assembly steps, eliminates particulates formed by secondary forming operations and eliminates the possibility of separation of components by bond failure possible with all previous devices.

## Description

1. Field of the Invention. This invention relates to a needle and more particularly to a needle assembly including a one-piece molded stylet or obturator for occluding the tip and/or bore of the needle.

2. Background. In the medical arts, there often is need for a percutaneous puncture to be made with a large bore needle for introduction of a medicament or withdrawal of a biopsy specimen. In order to avoid plugging the needle bore with soft tissue during the penetration, a formed stylet is placed within the bore of the needle to occlude the tip and bore preventing the bore from being plugged by soft tissue. Another application of a device within the bore of a needle is to block fluid flow back through a needle which is being used to insert a catheter or implant a sensor. When the needle occluding device is used for this purpose it is called an obturator. The distinction made by those in the art is that a needle occluding device is considered a stylet when it occludes the opening in the tip of a needle to prevent clogging or coring; and an obturator when its function is to occlude the bore to prevent leakage.

The stylets known in the art can be metal or plastic. Generally for needles intended for bone biopsy, the stylet is metal. The tip of the stylet is generally ground concurrently with the point of the needle to provide a smooth surface coincident with the surface of the opening of the needle. Representative of this type of bone biopsy needle assembly is the needle assembly taught in United States Patent 4,922,602 to Mehl.

Another bone biopsy needle assembly is taught in United States Patent 4,655,226 to Lee, which teaches a metal cannula having within it a straight solid needle with a bevelled distal end and a bent proximal end within a molded plastic cap which functions as a removable stylet.

United States Patent 4,838,282 to Netsch et al. teaches a bone biopsy needle with a stylet. The stylet is metal, having a sharpened distal tip and a thermoplastic handle being insert molded onto the proximal end of the stylet.

United States Patent 4,258,722 to Jesles et al. teaches a bone marrow sampling needle utilizing a metal cannula and stylet structure to close the adjacent distal end of the needle structure. The stylet has a proximal end extending from the corresponding proximal end or the needle structure which is lockable thereto to block relative rotation therebetween.

The same need to occlude the opening in a point applies to anesthesia needles intended for administration of epidural anesthesia. The needles are intended for introduction of anesthetic medicaments into the epidural space. The soft tissue, that must be penetrated before reaching the desired location of administration of the anesthesia, can potentially cause a clog in the bore of the needle or result in transfer of tissue into the epidural space. To preclude this, common practice dictates the use of a stylet to occlude the opening at the distal end of the needle.

A known needle assembly for administering epidural anesthesia incorporates a stylet composed of an extruded polyester tere-isophthalate rod which is adhesively bonded to a premolded acrylonitrile butadiene styrene (ABS) hub using epoxy. Other commonly used designs have stylets composed of extruded nylon, high density polyethylene or polypropylene rods insert molded into a polypropylene hub. Another available needle assembly makes use of a solid steel wire stylet epoxy adhesive bonded into a thermoplastic molded hub or handle.

In all of the examples cited, the metal or plastic rod must be trimmed or formed after insertion into the cannula. This trimming or forming leads to generation of undesirable particulates or, in the case of plastic, fine strands, which must he removed from the assembly prior to packaging and sterilization. Further, each of the prior art examples cited are multicomponent, having separate handles and shafts which are assembled in the manufacturing process. Of necessity, this includes multiple steps for assembly, tip forming, disassembly, cleaning and reassembly. The multiplicity of steps increases the complexity of the manufacturing process, likely reducing the yield of acceptable product. The possibility of generating particulates during the tip forming steps increases the likelihood of having undesirable particulates present in the final product. Further, by forming both the rod and the handle integrally, the possibility of separation by failure of an attachment bond is eliminated.

Although the prior art provides many unique stylet and obturator structures, there is still a need for a stylet or obturator with an integrally molded unitary structure comprising an elongate shaft and handle. The production of a stylet either in a ready-to-use one-step molding operation with an integrally formed tip or in a ready to insert and trim state, also formed by injection molding, represents a significant advance in simplification of the production process and enhances the integrity by eliminating the bonding of the shaft to the handle. The instant invention also provides a ready-to-use one-piece stylet capable of being manufactured using reduced number of manufacturing steps, but a real product improvement by eliminating the chance for incomplete removal of the particulates generated in the trimming process and eliminating the steps and associated problems encountered in the manufacture of two piece stylets.

### SUMMARY OF THE INVENTION

The invention is a needle assembly having a tip and/or lumen occluding device for a cannula to prevent the lumen from being blocked by soft tissue during a penetration when the device is a stylet, or backflow through the lumen when used as an obturator. The occluding device is an integrally molded unitary plastic structure. The device has a handle, and an elongate shaft having a formed tip which is generally coincident with the surface of the opening at the point of a needle, as a stylet, or within the lumen, when used as an obturator. The integrally formed handle of the lumen occluding device includes an integrally formed interlocking alignment means coaxially aligned with the formed tip of the device which removably conjugates with a corresponding structure on the hub of a needle coaxially aligned with the point of the needle. When the handle alignment means is engaged with its conjugate on the needle hub, the formed tip of the device and the surface of the needle point are generally coincident, and coaxial rotation of the device within the needle bore, which would defeat the tip coincidence with the surface of the needle point, is prevented.

### BRIEF DESCRIPTION OF THE DRAWINGS

Fig. 1 is a side elevation of a needle assembly of the present invention having a stylet within the lumen of the needle;
Fig 2 is a side elevation of the unitary integrally molded stylet of Fig. 1;
Fig. 3 is an enlarged proximal cross-sectional view of the needle assembly of Fig. 1 taken along line 3-3;
Fig. 4 is an enlarged side elevation view of the distal end of the needle assembly of Fig. 1;
Fig. 5 is a partial cross-sectional view of the formed distal end of the needle assembly of Fig. 1 along line 5,5 with the stylet removed;
Fig. 6 is a cross-sectional view of the distal end of the needle assembly of Fig. 1 along line 5,5 with a stylet in the lumen of the needle;
Fig. 7 is a side elevation of another embodiment of a needle assembly of the present invention having a stylet and wherein the needle is an epidural needle;
Fig. 8 is a partial cross-sectional view of the formed distal point of Fig. 8 with the lumen of the needle empty;
Fig. 9 is a partial cross-sectional view of the formed distal point of Fig. 7 with a stylet of the present invention in the lumen of the needle;
Figs. 10a and 10b are partial cross-sectional views of the formed angled point at the proximal end of a standard (a) needle and an epidural (b) needle with a stylet of the present invention having an elongate shaft extending through and beyond the opening in the point of the needle;
Fig. 11 is a partial cross-sectional view of a needle with a formed angled point at the proximal end of the needle wherein the lumen occluding device is an obturator having a rounded tip within the lumen of the needle; and
Fig. 12 is a representation of the multicomponent prior art stylet in unassembled form.

### DETAILED DESCRIPTION OF THE DRAWINGS

While this invention is satisfied by embodiments in many different forms, there is shown in the drawings and will herein be described several embodiments of the invention with the understanding that the present disclosure is to be considered descriptive of the principles of the present invention and is not intended to limit the scope of the invention to the embodiments illustrated. The scope of the invention will be measured by the appended claims and their equivalents.

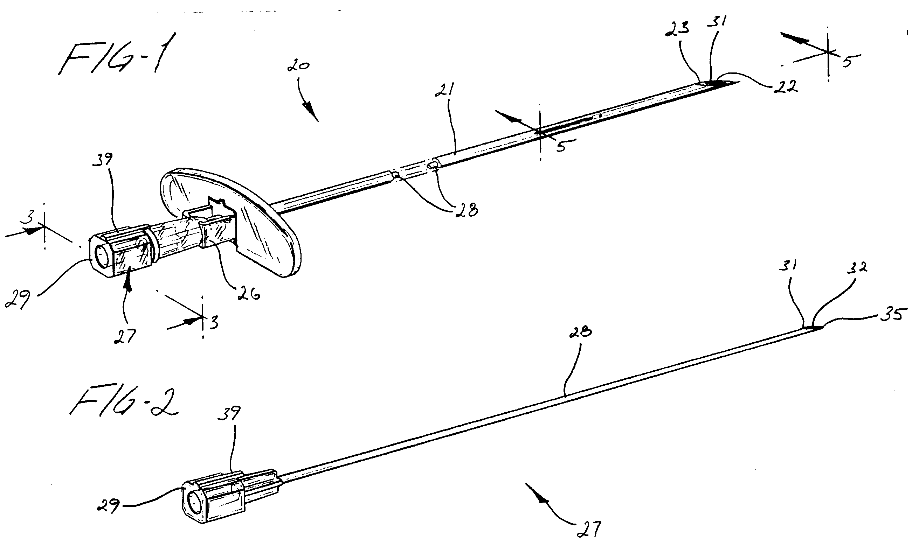

Adverting to Figs. 1-6, a needle assembly 20 comprises a needle 21 having a distal formed angled point 23 and a proximal end with a hub 26 connected thereto. The needle assembly includes a lumen occluding device 27 within it. The lumen occluding device 27 has an elongate shaft 28 removably positioned within a lumen 22 of the needle. The shaft 28 has a distal formed tip 31 with a distal tip surface 32. A periphery 35 of a tip surface 32 is generally coincident with a surface S defined by an opening 24 of the formed angled point 23 at the distal end of needle 21.

Lumen occluding device 27 has a handle 29 at its proximal end. The needle assembly 20 includes means for removably interlocking the hub 26 and the handle 29 to maintain a coaxial alignment between the formed distal tip 31 of the lumen occlusive device 27 and point 23 of needle 21. In this embodiment, means for interlocking includes projection 39 on handle 29 and a conjugate recess 38 formed in a bore 25 in hub 26.

Handle 29 of device 27 is a unitary structure integrally injection molded with the elongate shaft 28 and the formed tip 31 having the surface 32 formed in the unitary molding operation. Device 27 has a parting line 48 from the injection molding process. The parting line 48 is substantially parallel to at least a portion of a linear axis 47 of shaft 28 of the device 27.

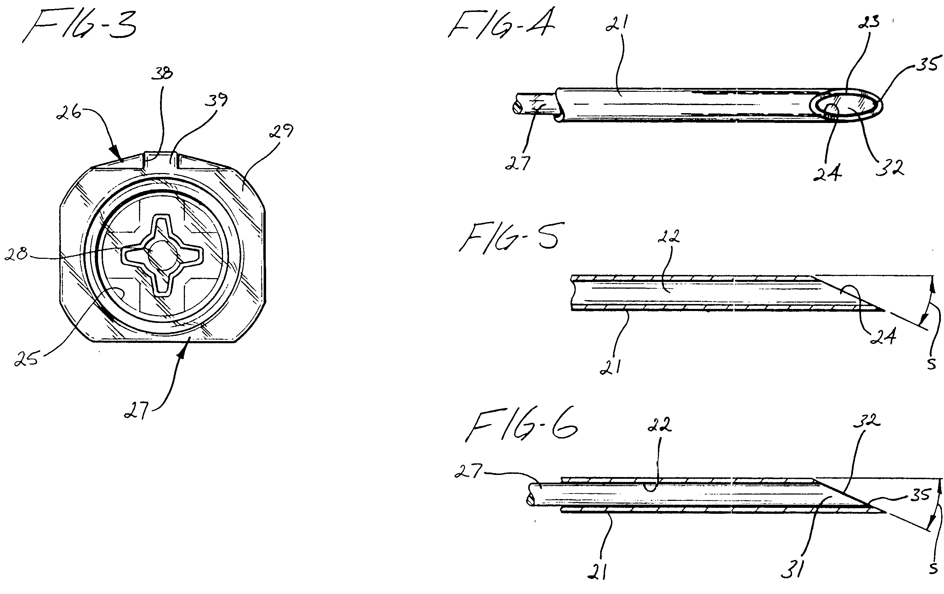

Surface 32 of the distal tip 31 of device 27 is molded so that when device 27 is inserted into the needle lumen 22, periphery 35 of surface 32 and the surface S described by distal point 23 at needle opening 24 are substantially coincident with each other as illustrated in Fig. 6. This coincidence serves to occlude opening 24 and prevents clogging of lumen 22 by soft tissue during a percutaneous penetration. It is desirable that the substantial coincidence be within 0.007 inches and preferably within +/- 0.002 inches.

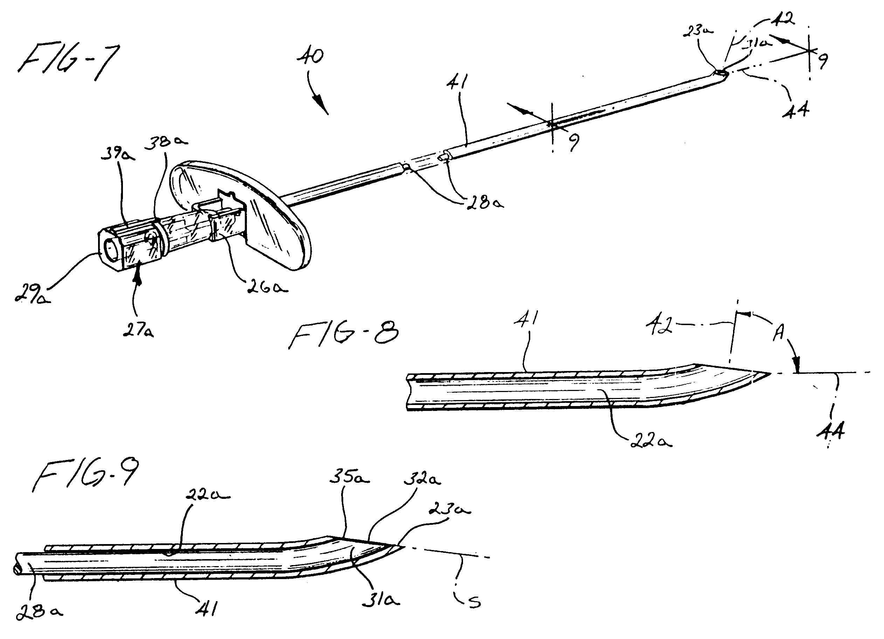

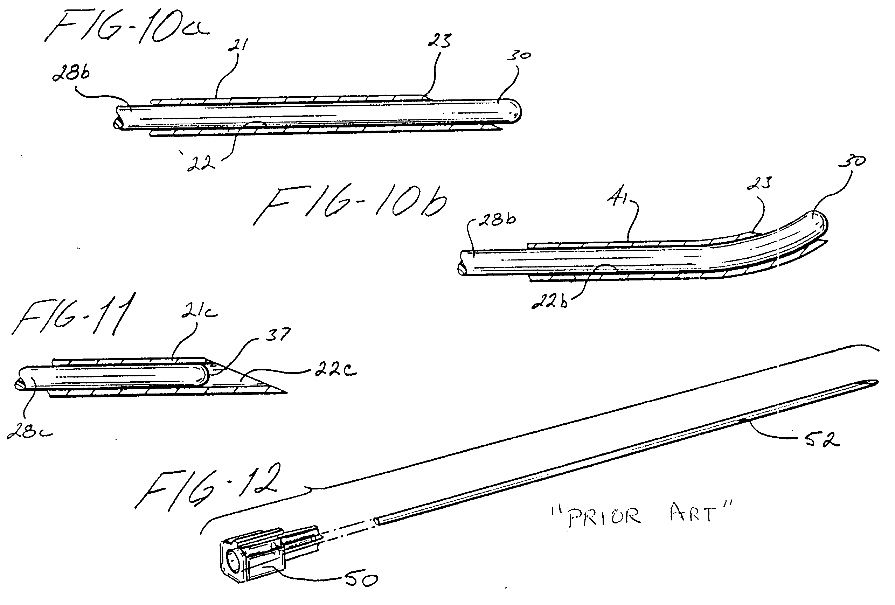

An alternate embodiment of the instant invention is shown in Figs. 7,8 and 9 wherein the needle 41 of a needle assembly 40 is an epidural needle for introduction of a medicament into the epidural space. In this alternative and other alternate embodiments contained in the instant specification, elements similar in structure to the needle assembly of the embodiment of Figs. 1-6 are shown. Accordingly, substantially similar components that perform substantially similar functions will be numbered identically to those components of the embodiment of Figs. 1-6, except a suffix "a" will be used to identify those components in Figs. 7-9, suffix "b" will be used to identify similar components in Figs. 10a and 10b and suffix "c" will be used to identify similar components in Fig. 11.

Adverting to Figs. 7,8 and 9, epidural needle 41 has a bend with an axis 42 forming an angle A at the distal tip 23a with a longitudinal axis 44 of the needle. The angle A is 35° or less. When a stylet of the present invention 27a having a handle 29a, an elongate shaft 28a and a distal formed tip 31a is fully inserted into the needle lumen 22a so that a conjugating interlock projection 39a on the handle 29a is fully engaged with a conjugate recess portion 38a in a bore 25a in a hub 26a, a periphery 35a of the surface 32a of the distal tip 31a of device 27a and a surface Sa described by an opening 24a at a distal point 23a of the needle 41 are within 0.007 inches of coincidence with each other.

The design of stylet 27a when intended for use in the needle assembly 40 which contains needle 41 is selected so that the periphery 35a of surface 32a of formed distal tip 31a of device 27a is substantially coincident with surface Sa described by opening 24a at the distal point 23a of needle 41 when fully inserted into the lumen 22 of needle 41 and conjugating projection 39a and recess 38a are fully engaged. The design of tip 31a takes into account compliance of elongate shaft 28a with the bend with axis 42 of needle 41 to ensure substantial coincidence of periphery 35a with surface Sa defined by opening 24a.

Preferably, shaft 28a of device 27a has a diameter of less than 4 mm. and is from one to eight inches long. Device 27a is preferably injection molded in a mold tool from a thermoplastic resin, most preferably the resin is polypropylene. The resin can be compounded with a suitable agent to render it radiopaque. The shaft is flexible and can conform to a bend in a needle while retaining axial alignment between alignment means and tip surface.

Adverting to Figs. 10a and 10b, another alternate embodiment of the instant invention is shown, similar to elements of Figs. 1-6 and 7-8, except that the elongate shaft does not have a formed tip generally coincident with the surface of the point. The unitary integrally injection molded handle 29b and an elongate shaft 28b has the shaft length sized so that a tip 30 projects through and beyond the formed angled point 23b of the needle, whether a standard needle 21 (Fig. 10a) or an epidural needle 41 (Fig. 10b). A secondary trimming operation to form a substantially coincident tip is required, while retaining the other benefits inherent in the unitary integrally injection molded invention.

Adverting to Fig. 11, another embodiment of the device 27c is to function as an obturator in assembly 46, to occlude a needle lumen 22c of a needle cannula 21c. In this embodiment, an elongate shaft 28c has a rounded formed tip 37 integrally injection molded. The length of the shaft 28c is selected so that the tip 37 remains within the lumen 22c of the needle 21c.

Adverting to Fig. 12, until the present invention, all prior art stylets were manufactured by assembly from multiple independent components as shown. The separate injection molded handle 50 is bonded to an elongate shaft 52 by an insert molding or an adhesive bonding process. The elongate shaft 52 is generally formed by an extrusion process and cut to a desired length. A tip on the elongate shaft would then be formed to be generally coincident with the surface of the needle point by a trimming process when the assembled but untrimmed device is fully inserted into a needle. The excess elongate shaft projects through and beyond the opening in the point of the needle. This trimming process would then, of necessity, be followed by some type of cleaning process to remove any residue generated during the trimming. As described hereinabove, the prior art device is a multiple step process.

Several undesirable possibilities arise from the multiple step process, including damage to the already formed distal angled point of the needle in any of the additional steps; generation of particulates during the tip forming operation; need to dissemble the needle assembly for cleaning after the secondary tip forming operation; and danger of inadequate removal of particulates formed in the tip forming operation. Further, by eliminating the need to bond the shaft to the handle, any loss of product from inadequate bonding is prevented and product in-use failure by separation is eliminated.

It is well known in the manufacturing arts, that each step of a multistep operation has an impact on the overall yield of acceptable product produced, the more steps, the greater the likelihood of reduced yield of acceptable product. Thus, the present invention, wherein a stylet or obturator can be produced in one step ready-to-use, represents an advancement to the art not before accomplished.

## Claims

1. A needle assembly comprising:
a needle cannula having a proximal end and a distal end defining a longitudinal axis, a formed angled point at said distal end, and a lumen therethrough defining an opening therein at said distal end, said distal end defining a surface;
a hub at said proximal end of said cannula, said hub having a bore therethrough in fluid communication with said lumen;
a lumen occluding device having an elongate solid shaft positioned removably within and occluding said opening and said lumen, said shaft having a proximal end and a distal end defining a longitudinal shaft axis, said shaft having a diameter of less than 4 mm and a handle at the proximal end of said shaft; and
said lumen occluding device being integrally injection molded in a mold tool in a single step to form a unitary plastic structure, wherein a parting line of the tool is substantially parallel to a portion of said shaft longitudinal axis, and wherein said device has a formed tip at said distal end of said shaft.

2. The needle assembly of Claim 1 wherein said lumen occluding device formed tip has a distal surface substantially coincident with said surface defined by said opening.

3. The needle assembly of Claim 2, wherein a periphery of said distal tip surface is substantially within 0.18 mm of said surface defined by said opening.

4. The needle assembly of Claim 1, wherein said device is molded from a thermoplastic resin.

5. The needle assembly of Claim 1, wherein said lumen occluding device includes an integrally molded rounded tip at its distal end, said tip being contained within said lumen.

6. The needle assembly of Claim 1, wherein said needle is an epidural anesthesia needle, having a bend in a direction at said distal end, said direction is generally 35° or less from said longitudinal axis;
said elongate shaft of said lumen occluding device flexibly conforms to said bend in said needle;

7. The needle assembly of Claim 6, wherein said lumen occluding device has a formed tip at said distal end of said shaft, said tip having a distal surface being substantially coincident with said surface defined by said opening.

8. The needle assembly of Claim 7, wherein a periphery of said distal tip surface is within substantially 0.18 mm of the surface defined by said opening.

9. The needle assembly of Claim 8, wherein the needle hub and the device handle have a separable conjugating interlocking means for providing a coaxial alignment of said shaft and said cannula.

10. A method of manufacturing a needle assembly comprising:
providing a needle cannula having a proximal end and a distal end defining a longitudinal axis, a lumen therethrough and a formed angled point defining an opening therein at said distal end, said opening defining a surface;
providing a needle hub at the proximal end of said cannula, said hub having a bore therethrough in fluid communication with said lumen;
molding a lumen occluding device having an elongate shaft and a handle, said shaft having a proximal end and a distal end defining a longitudinal axis, said shaft having a diameter of less than 4 mm with a length of about 2 to 20 cm, said handle being at said proximal end of said shaft, said lumen occluding device being integrally formed by injection molding in a single step in a mold tool to form a unitary plastic structure, wherein a parting line of said mold tool is substantially parallel to a portion of said longitudinal shaft axis, said shaft including a formed point at its distal end having a distal surface;
providing a separable conjugating interlocking means on said handle and said hub for providing a coaxial alignment of said point at said distal end of said cannula and said tip of said device;
placing said device within said lumen; and
engaging said interlocking means on said handle and said hub so that said distal surface and said surface defined by said opening of said needle point are substantially coincident.
